# EUROPEAN PATENT APPLICATION

(11) **EP 1 434 054 A1**
(43) Date of publication of application: **30.06.2004**
(21) Application number: 03258202.5
(22) Date of filing: 23.12.2003
(51) Int. Cl.: G01N 33/92, C12Q 1/60

(54) **Biosensor for determining low density cholesterol**

(30) Priority: 25.12.2002 JP 2002375101
(71) Applicant: MATSUSHITA ELECTRIC INDUSTRIAL CO., LTD., Kadoma-shi, Osaka 571-8501 (JP)
(72) Inventor: Watanabe, Motokazu, Toyonaka-shi Osaka, 560-0025 (JP); Nakaminami, Takahiro, Toyonaka-shi Osaka, 560-0033 (JP); Taniije, Yuko, Osaka-shi Osaka, 542-0066 (JP); Miyashita, Mariko, Nishinomiya-shi Hyogo, 663-8032 (JP); Yoshioka, Toshihiko, Hirakata-shi Osaka, 573-0035 (JP)
(74) Representative: Price, Paul Anthony King

(57) **Abstract**

The sensor is capable of accurately and simply measuring LDL cholesterol with only a one-time supply of a sample. A substrate of the biosensor carries cholesterol esterase, cholesterol oxidase or cholesterol dehydrogenase, a reagent for bringing a selective enzyme reaction with LDL, peroxidase, and a dye source.

## Description

### Background of the invention

The present invention relates to a biosensor for use in determination of cholesterol contained in low-density lipoprotein (LDL) in a sample to be detected e.g. blood, serum and plasma. In particular, the present invention relates to a structure of a biosensor capable of measuring said cholesterol in a single step.

Cholesterol concentrations in LDL have recently been recognized as important in diagnoses of high-cholesterol thrombus. LDL cholesterol of this sort has hitherto been determined by fractionation by the use of ultracentrifugation. This method however requires a specialized particular device and further raises a problem of necessitating long hours of measurement.

A typical determination method where the ultracentrifugation is not conducted is a method of determining the respective concentrations of total cholesterol, high-density lipoprotein (HDL) cholesterol and triglyceride in a sample, and then calculating an LDL cholesterol concentration by means of the Friedewald formula. This method has a problem, however, that in using a sample to be detected which contains a high value of triglyceride, reliability in terms of reproducibility as well as accuracy is low.

Methods as described below have been proposed in recent years as determination methods of cholesterol in a low-density lipoprotein, requiring no triglyceride value.

Firstly, there is for example described in Clinical Chemistry, 1998, Vol. 44, Page 522 a method of oxidizing only cholesterol in low-density lipoprotein with an enzyme, in the presence of α-cyclodextrin sulfate, dextran sulfate, magnesium ions and polyoxyethylene-polyoxypropylene block copolyether, to determine a cholesterol concentration in the low-density lipoprotein, from a degree of dye color.

In the above method, the reactivity of the enzyme to cholesterol in chylomicron, as well as cholesterol in very-low-density lipoprotein (VLDL), in a sample to be detected is reduced by α-cyclodextrin sulfate, dextran sulfate and magnesium ions, and further, the reactivity of the enzyme to cholesterol in high-density lipoprotein in the sample to be detected is reduced by polyoxyethylene-polyoxypropylene block copolyether.

Secondly, there is for example proposed in Japanese Laid-Open Patent Publication No. Hei 10-84997, a method of oxidizing only cholesterol in low-density lipoprotein with an enzyme, in the presence of an amphoteric surfactant and aliphatic amines having a carboxyl group or a sulfone group, to determine a cholesterol concentration in the low-density lipoprotein, from a degree of dye color.

The above method is to reduce the reactivity of the enzyme to cholesterol contained in other sorts of lipoprotein than low-density lipoprotein in the presence of the amphoteric surfactant.

Thirdly, there is for example proposed in U. S. Patent No. 4,185,963 a patent document 2 a method of oxidizing only cholesterol in low-density lipoprotein with an enzyme by treating a sample to be detected with polycation, to determine a cholesterol concentration in the low-density lipoprotein, from a degree of dye color.

Fourthly, for example, in a method described in U. S. Patent No. 5,401,466, high-density lipoprotein is absorbed by porous silica, and then an insoluble complex of the absorbed high-density lipoprotein, chylomicron and very-low-density lipoprotein is formed by polyanion/bivalent cation. After this complex is removed from a solution as a precipitate, only cholesterol in low-density lipoprotein is oxidized with an enzyme, to determine a cholesterol concentration in the low-density lipoprotein, from a degree of dye color.

It is possible to determine a value of low-density lipoprotein cholesterol, according to the aforesaid methods, even with the use of a sample to be detected having a high value of triglyceride.

### Brief summary of the invention

Nevertheless, there is a disadvantage in the first to fourth methods that, in order to conduct a highly reproducible and accurate measurement, an operation is made complicated, necessitating preparation of a first reagent aqueous solution and a second reagent aqueous solution, and accurate addition of a prescribed amount of each of the first reagent aqueous solution and the second reagent aqueous solution to a serum sample for a certain period of time.

In view of the conventional problems as thus described, accordingly, an aspect of the present invention is to provide a biosensor capable of accurately and simply measuring cholesterol contained in LDL (LDL cholesterol) by a one-time supply of a sample, while not requiring addition of two sorts or more of reagents to a sample to be detected in different timings, when a patient unskilled in addition of a reagent uses the biosensor. Particularly, an aspect of the present invention is to provide a biosensor allowing a user to conduct a highly reproducible and accurate determination, with no maintenance performed.

The present invention relates to a biosensor comprising:
a substrate; and
cholesterol esterase, cholesterol oxidase or cholesterol dehydrogenase, a reagent for bringing a selective enzyme reaction with low-density lipoprotein, peroxidase, and a dye source, which are dryly carried on the substrate.

It is preferable in the biosensor that the biosensor further comprises a carrier on the substrate and that the carrier be made of a paper filter, a glass filter, a membrane filter or a cellulose fiber.

It is also preferable that the cholesterol esterase, the cholesterol oxidase or cholesterol dehydrogenase, the reagent, the peroxidase, and the dye source be carried on the carrier by freeze drying, high-temperature drying, hot-air drying or natural drying.

In the biosensor in accordance with the present invention, the cholesterol esterase, the cholesterol oxidase or cholesterol dehydrogenase, the reagent for bringing a selective enzyme reaction with low-density lipoprotein, peroxidase, and the dye source may be contained as in the state of a reagent dried body. This reagent dried body is a solid product obtained by dropping a solution containing a reagent and the like, followed by drying.

In the present invention, a plurality of the solid reagent dried bodies can be provided in the biosensor, and each of the cholesterol esterase, the cholesterol oxidase or cholesterol dehydrogenase, the reagent for bringing a selective enzyme reaction with low-density lipoprotein, peroxidase, and the dye source may be contained in any of the reagent dried bodies. The shape of the reagent dried body is not particularly limited but may be pellet, disk, cube, rectangular parallelepiped, plate or the like.

It is preferable that the reagent be carried as separated from the cholesterol esterase, the cholesterol oxidase or cholesterol dehydrogenase, and the dye source.

It is preferable that the biosensor further comprises a flow channel and that the reagent be provided upstream of the flow channel, while the cholesterol esterase, the cholesterol oxidase or cholesterol dehydrogenase, the peroxidase, and the dye source be provided downstream of the flow channel.

It is preferable that the reagent be a surfactant.

It is preferable that the surfactant be either a nonion-type surfactant or an anion-type surfactant.

It is also preferable that the nonion-type surfactant be a nonion-type polymer.

It is preferable that the biosensor further comprises α-cyclodextrin sulfate.

It is preferable that the biosensor further comprises dextran sulfate.

It is preferable that the biosensor further comprises a pH buffer.

It is preferable that the biosensor further comprises a bivalent metal salt. The bivalent metal salt is preferably any of magnesium salt, calcium salt, and manganese salt.

It is preferable that the biosensor further comprises lipoprotein lipase.

It is preferable that the biosensor further comprises a filter for separating a specific component from a sample.

It is also preferable that the passage of the sample through the filter and gravity be parallel, vertical or oblique in direction.

The present invention further relates to a test system, including the aforesaid biosensor and a first light source.

It is preferable that this test system further includes a second light source.

A sample can be detected by either the first light source or the second light source. Moreover, the information from the first light source can be corrected or compensated by the information from the second light source.

The use of the biosensor in accordance with the present invention allows determination of cholesterol in LDL with high reproducibility and accuracy by a one-time supply of a sample. Not necessitating a complex operation, a determination method using the biosensor in accordance with the present invention is well suited for LDL cholesterol measurement kits to be used in places other than specialized institutions such as hospitals and clinical laboratories.

Namely, the use of the biosensor in accordance with the present invention enables accurate and simple determination of LDL cholesterol by only a one-time supply of a sample to be detected without addition of two sorts or more of reagents to a sample to be detected in different timings, even when a patient unskilled in addition of a reagent uses the biosensor.

Furthermore, the biosensor in accordance with the present invention has the advantage of not deteriorating even when stored since a reagent has been dried because water contained in a reagent layer, especially a reagent layer containing enzymes such as cholesterol oxidase and peroxidase, is removed sufficiently, and also has the merit of being maintenance-free.

### Brief description of the several views of the drawings

FIG. 1 is an exploded oblique view of a biosensor in accordance with a first embodiment of the present invention.
FIG. 2 is an exploded oblique view of a biosensor in accordance with a second embodiment of the present invention.
FIG. 3 is an exploded oblique view of a biosensor in accordance with a third embodiment of the present invention.
FIG. 4 is an exploded oblique view of a biosensor in accordance with a fourth embodiment of the present invention.
FIG. 5 is an exploded oblique view of a biosensor in accordance with a fifth embodiment of the present invention.
FIG. 6 is a flowchart of a measurement using a test system including the biosensor in accordance with the present invention.

### Detailed description of the preferred embodiments

In order to accomplish the aforesaid objectives, the present invention provides a biosensor composed of a dry-type test strip comprising:
a substrate; and
cholesterol esterase, cholesterol oxidase or cholesterol dehydrogenase, a reagent for bringing a selective enzyme reaction with low-density lipoprotein, peroxidase, and a dye source, which are all carried on the substrate.

It is preferable that the biosensor further comprise a carrier and that the cholesterol esterase, the cholesterol oxidase or cholesterol dehydrogenase, the reagent, the peroxidase, and the dye source be dryly carried either on the carrier or in the carrier.

It is preferable that the carrier be selected from the group consisting of a paper filter, a glass filter, a membrane filter and a cellulose fiber. It is to be noted that a carrier used with an aim to dryly carry a reagent may serve to separate hemocytes. It should also be noted that a mesh may further be provided. This carrier is held on the substrate.

Herein described is a principle of an LDL cholesterol measurement in the biosensor in accordance with the present invention, for instance. When a sample containing low-density lipoprotein (LDL), high-density lipoprotein (HDL) and very-low-density lipoprotein (VLDL) is introduced into the biosensor in accordance with the present invention, only LDL is solubilized by a reagent for bringing a selective enzyme reaction with LDL. Thereafter, cholesterol in LDL is reacted with cholesterol esterase to generate cholesterol, which is then reacted with cholesterol oxidase or cholesterol dehydrogenase to generate cholestenone and hydrogen peroxide (H₂O₂).

Subsequently, the resultant hydrogen peroxide is reacted, for example, with 4-aminoantipyrin, HDAOS and the like, in the presence of peroxidase, to generate quinone dye. Measuring the maximum absorption value (583 nm) of this quinone dye allows determination of LDL cholesterol in the sample.

As the dye source mentioned can be 4-aminoantipyrin, phenol, 3-hydroxy-2,4,6-triiodo benzoic acid, [3-bis(4-chlorophenyl)methyl-4-dimethylaminophenyl]amine (BCMA), or the like.

Further added may be such a Trinder reagent as N-ethyl-N-(2-hydroxy-3-sulfopropyl)-3,5-dimethoxyaniline (DAOS), N-(2-hydroxy-3-sufopropyl)-3,5-dimethoxyaniline (HDAOS), N-ethyl-N-(2-hydroxy-3-sulfopropyl)-3,5-dimethylaniline (MAOS), N-ethyl-N-(3-methylphenyl)-N'-succinyl-ethylenediamine (EMSE) or N-ethyl-N-(2-hydroxy-3-sulfopropyl)-m-toluidine (TOOS). Among those Trinder reagents preferred is HDAOS as being resistant to influences from other elements in the sample. As for the color source for chemically generating a light, luminol, isoluminol or the like can be used.

There is no particular limitation to the material for the substrate on which the carrier is held, so long as it has a certain degree of rigidity. For example, a thermoplastic resin such as polyethylene, polystyrene, poly vinyl chloride, polyamide or saturated polyester, or a thermosetting resin such as a urea resin, a melamine resin, a phenol resin, an epoxy resin or an unsaturated polyester resin can be mentioned.

The dryly carrying method may be a conventional method e.g. freeze drying, high-temperature drying, hot-air drying and natural drying. The freeze drying is preferred for the reason of having excellent stability in storing a reagent while the high-temperature drying or the hot-air drying is preferred for the reason of requiring short drying time.

It is preferable that the reagent for bringing a selective enzyme reaction with LDL be provided as separated from the cholesterol esterase, the cholesterol oxidase or cholesterol dehydrogenase, the peroxidase, and the dye source. This is because the separation enables prevention of the reagent from exerting an influence on the cholesterol esterase and the cholesterol oxidase or cholesterol dehydrogenase, during storing of the biosensor.

In the biosensor in accordance with the present invention, it is further preferable that, for example, a flow channel be provided in the substrate, and the reagent for brining a selective enzyme reaction with LDL be provided upstream of the flow channel, while the cholesterol esterase, the cholesterol oxidase or cholesterol dehydrogenase, the peroxidase, and the dye source be provided downstream of the flow channel. This is because even higher selectivity for LDL can be exerted by first subjecting a sample such as blood or plasma to the sufficient action of the reagent, and then bringing an enzyme reaction by cholesterol esterase and cholesterol oxidase.

The reagent for bringing a selective enzyme reaction with LDL is preferably a surfactant. It is preferable that the surfactant be either a nonion-type surfactant or an anion-type surfactant. It is also preferable that the nonion-type surfactant be a nonion-type polymer.

It should be noted that polyoxyethylene alkyl ether or polyoxyethylene alkyl aryl ether is preferred as the nonion-type surfactant, while a polyoxyehylene-polyoxypropylene copolymer is preferred as the nonion-type polymer.

Further, sodium lauryl sulfate is preferred as the anion-type surfactant.

It is preferable that the biosensor in accordance with the present invention further comprises α-cyclodextrin sulfate. In this case, it may be included in a carrier such as a glass filter, as in the case with the reagent for bringing a selective enzyme reaction with LDL. Other than α-cyclodextrin sulfate used may be α-cyclodextrin, β-cyclodextrin, γ-cyclodextrin, or a cyclodextrin derivative, such as α-cyclodextrin sulfate, β-cyclodextrin sulfate, dimethyl-β-cyclodextrin or trimethyl-β-cyclodextrin. Dextran sulfate may further be added.

A pH buffer may further be added to the biosensor in accordance with the present invention for the purpose of effectively enhancing the action by the reagent for bringing a selective enzyme reaction with LDL, and the reaction by the enzyme like cholesterol oxidase. In this case, the pH buffer may be included in a carrier such as a glass filter as in the case with the enzyme like cholesterol oxidase. As the pH buffer mentioned can be phthalate, maleate, succinate, phosphate, acetate, borate, citrate, glycine, tris(hydroxymethyl)aminomethane (Tris), or the like. Further preferred is a Good's buffer such as 2-(N-morpholino)ethane sulfonic acid (MES), piperazine-N,N'-bis(2-ethane sulfonic acid) (PIPES), 3-(N-morpholino)propane suofonic acid (MOPS) or N-2-hydroxyethyl piperazine-N'-2-ethane sulfonic acid (HEPES), since those reagents can adjust pH to be about neutral.

The biosensor in accordance with the present invention may further be added with a bivalent metal salt in order to improve selectivity for LDL. In this case, it may be included in a carrier such as a glass filter as in the case with the enzyme like cholesterol oxidase. Herein, magnesium salt, calcium salt or manganese salt is preferred as the bivalent metal salt. Among them most preferred is magnesium salt for the purpose of improving selectivity for LDL. The biosensor may further comprise lipoprotein lipase.

Moreover, the biosensor in accordance with the present invention further comprises, in addition to a filter constituting the aforesaid carrier, a second filter into which hemocytes in a sample may be separated. As the filter mentioned can be a glass filter, a paper filter, a membrane filter, a mesh or cotton.

The aforesaid biosensor constitutes a test system in combination with a first light source. The test system may further include a second light source. In this case, the information from the first light source can desirably be corrected or compensated by the information from the second light source. Further, a sample can be detected by means of the first light source or the second light source.

While a sample to be detected may be any body fluid, for example, blood, plasma, serum, an interstitial fluid or the like can be used.

Next, preferable examples of the structure of the biosensor in accordance with the present invention are described with reference to drawings.

### Embodiment 1

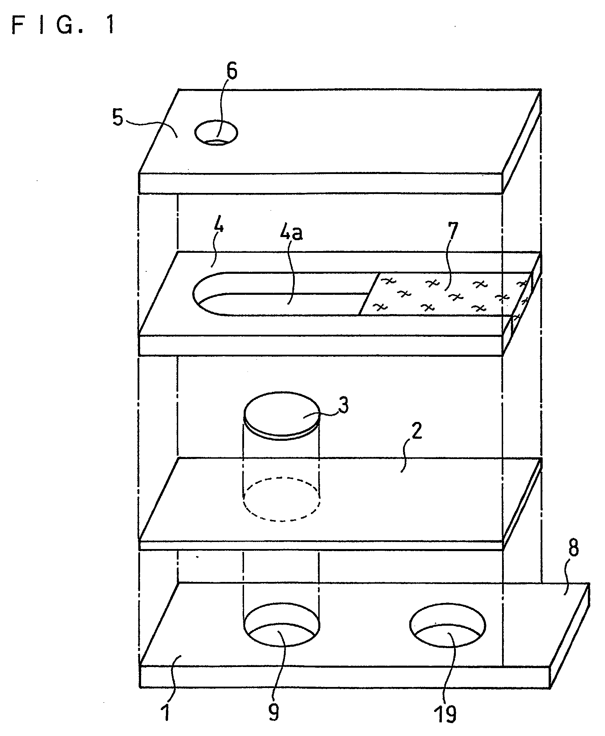

FIG. 1 is an exploded oblique view of a biosensor in accordance with a first embodiment of the present invention. This biosensor is constituted of a substrate 1 having two through holes (apertures) 9 and 19, a transparent sheet 2, a reagent dried body 3, a spacer 4 having a slit 4a, a filter 7 for filtering hemocytes from blood, and a cover 5 having an air vent 6.

The air vent 6 is located at the left end of a slit 4a of the spacer 4, and a filter 7 is provided in the right half of the slit 4a. The reagent dried body 3 is provided on the transparent sheet 2 so as to be located in the slit 4a after the assembly. The reagent dried body 3 is located over the hole 9, and the hole 19 is located as opposed to the filter 7. The substrate 1 is longer than the transparent sheet 2, the spacer 4 and the cover 5 in terms of the respective right sides thereof, so as to form a sample supply portion 8 to be supplied with a sample.

In the case of using the biosensor having such a structure as shown in FIG. 1, a sample such as blood is dropped onto the vicinity of the sample supply portion 8. Hemocytes are filtered from the supplied blood while the blood flows in a horizontal direction within the filter 7. The filtered blood reaches the vicinity of the air vent 6 to dissolve the reagent dried body 3. At this time, a reagent for bringing a selective enzyme reaction with LDL, which exists in the filter 7, and cholesterol esterase, cholesterol oxidase, peroxidase, 4-aminoantipyrin and HDAOS in the reagent dried body 3 act, resulting in coloring. The degree of the color is measured by irradiating a light, emitted by a light-emitting diode for instance, from the under part of the substrate 1 through the hole 9 as the measurement portion and then detecting a reflected light by a photodiode.

It is possible by the method thus described to conduct a one-step measurement of LDL cholesterol in blood by only a one-time supply of blood. According to the biosensor in accordance with the present embodiment, there is no need for a complex operation of separately adding samples in certain timings, thereby enabling a highly reproducible and accurate determination. Herein, with the reagent having been dried, the biosensor has the advantage of not deteriorating even when stored for a long period of time, since water contained in a reagent layer, especially a reagent layer comprising cholesterol oxidase etc. and peroxidase etc., is removed sufficiently, and requires no maintenance.

It is to be noted that introduction of a sample, or the sort of sample, may be detected by measuring lights by means of a light-emitting diode and a photodiode. It should also be noted that a sample may be detected by measuring a light from the hole 19 by the use of the second light source. Further, the information provided according to the measurements of lights by the light-emitting diode and the photodiode may be corrected or compensated by the information provided according to the measurement of a light by the use of the second light source.

### Embodiment 2

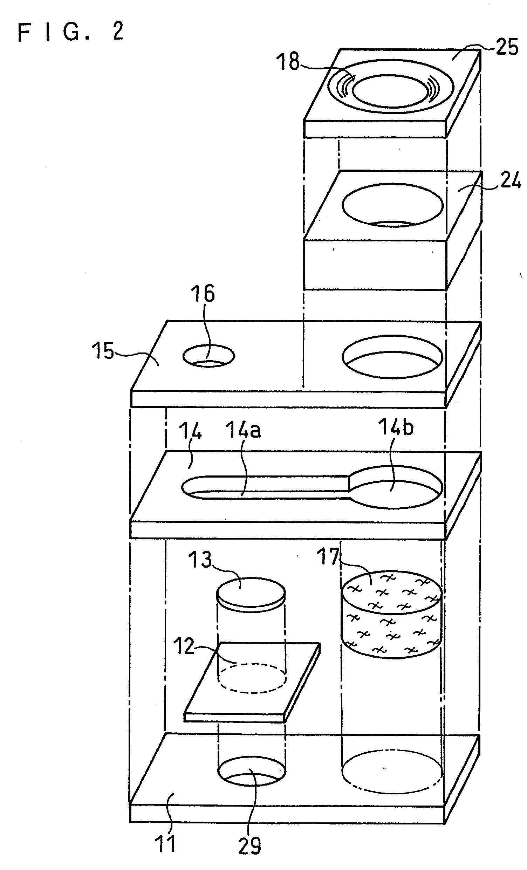

Next, FIG. 2 is an exploded oblique view of a biosensor in accordance with a second embodiment of the present invention. This biosensor is constituted of a substrate 11 having a through hole 29, a transparent sheet 12, a reagent dried body 13, a spacer 14 having a slit 14a, a filter 17 for filtering hemocytes from blood, a cover 15 having an air vent 16, a spacer 24 whose height is adjustable to consist with the height of the filter 17, and a cover 25 having a mortar(bowl)-shaped sample supply portion 18.

The air vent 16 is located at the left end of the slit 14a of the spacer 14, and the right half of the slit 14a is provided with an opening portion 14b that can accommodate the filter 17. The reagent dried body 13 is provided on the transparent sheet 12 so as to be located in the slit 14a after the assembly. The reagent dried body 13 is located over the hole 29.

On the right-hand side of the substrate 11 held is the filter 17, which is fitted in the opening portion 14b of the spacer 14 and the opening portion 16b of the cover 15. A spacer 24 and a cover 25 are then provided above the opening portion 16b of the cover 15, and a sample can be introduced from the sample supply portion 18.

In the case of using the biosensor having such a structure as shown in FIG. 2, a sample such as blood is dropped into the sample supply portion 18. Being in mortar shape, the sample supply portion 18 helps with a smooth sample supply. Hemocytes are filtered from the supplied blood while the blood flows in a vertical direction within the filter 17. Hereat, a reagent present in the filter 17 acts to bring a selective enzyme reaction with LDL.

In the biosensor, the filtered blood flows horizontally to the vicinity of the air vent 16, to dissolve the reagent dried body 13. At this time, the reagent for bringing a selective enzyme reaction with LDL, which exists in the filter 17, and cholesterol esterase, cholesterol oxidase, peroxidase, 4-aminoantipyrin and HDAOS in the reagent dried body 13 act, resulting in coloring as in Embodiment 1.

It is possible by the method thus described to measure LDL cholesterol in blood in a single step. Furthermore, according to the biosensor in accordance with the present embodiment, there is no need for separate addition of reagents in certain timings, and no need for maintenance. There is moreover an advantage that the measurement time is reduced since a sample such as blood flows vertically within the filter through the use of gravity.

### Embodiment 3

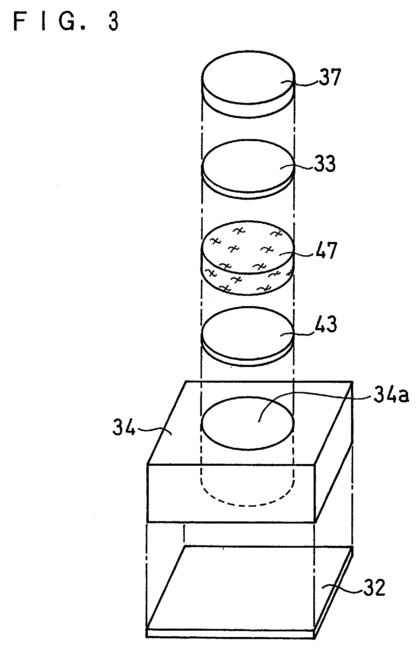

Next, FIG. 3 is an exploded oblique view of a biosensor in accordance with a third embodiment of the present invention. This biosensor is constituted of a spacer 34 having a through hole 34a, a transparent sheet 32, a reagent dried body 43, a filter 47, a reagent dried body 33 for bringing a selective enzyme reaction with LDL, and a filter 37 for filtering hemocytes from blood.

This biosensor does not comprise a substrate, and the reagent dried body 43, the filter 47, the reagent dried body 33 and the filter 37 are laminated in the described order to be accommodated in the hole 34a of the spacer 34 provided on the transparent sheet 32.

In the case of using the biosensor having such a structure as shown in FIG. 3, a sample such as blood is dropped onto the filter 37. Hemocytes are filtered from the supplied blood while the blood flows in a vertical direction in the filter 37 or the filter 47. Hereat, the reagent dried body 33 acts to bring a selective enzyme reaction with LDL. The biosensor has the advantage of reducing the measurement time since a sample such as blood flows vertically within the filter through the use of gravity.

It is possible by the method thus described to measure LDL cholesterol in blood in a single step. According to the biosensor in accordance with the present embodiment, there is no need for separate addition of reagents in certain timings, and no need for maintenance.

Further, since the reagent for bringing a selective enzyme reaction with LDL has been separated, via the filter 47, from cholesterol esterase, cholesterol oxidase and the like, the reagent for bringing a selective enzyme reaction with LDL can be the first to act on the sample.

Favorably, the sample can be sufficiently acted upon by the reagent for bringing a selective enzyme reaction with LDL before being subjected to the action of cholesterol esterase, cholesterol oxidase and the like. Moreover, there is an advantage that, with lipoprotein lipase added, further improvement of accuracy as well as reduced measurement time can be expected.

### Embodiment 4

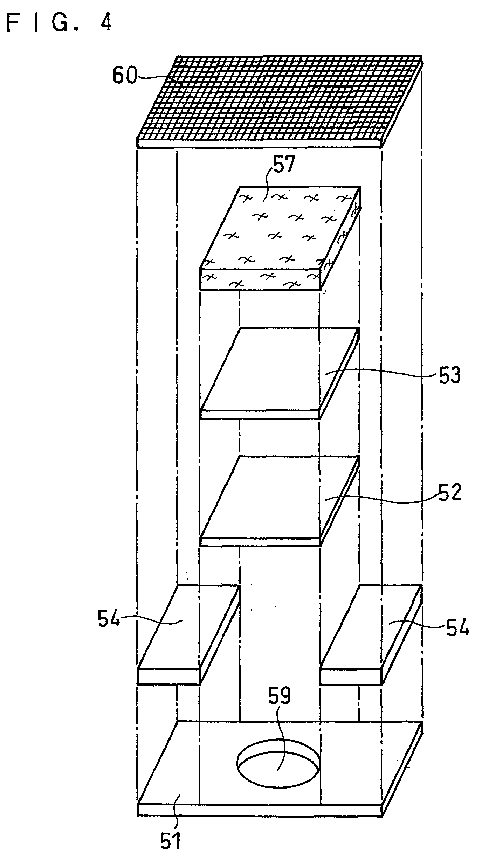

Next, FIG. 4 is an exploded oblique view of a biosensor in accordance with a fourth embodiment of the present invention. This biosensor is constituted of a substrate 51 having a through hole 59, a transparent sheet 52, two spacers 54, a reagent dried body 53, a filter 57 for filtering hemocytes from blood, and a mesh 60.

The through hole 59 is provided in the center of the substrate 51, and to the both sides thereof, the two spacers 54 are respectively provided. The transparent sheet 52, the reagent dried body 53 and the filter 57 are laminated in the described order on the hole 59, and the mesh 60 is disposed on the top of the laminate. The size of this mesh 60 is equivalent to that of the substrate 51.

In the case of using the biosensor having such a structure as shown in FIG. 4, a sample such as blood is dropped onto the mesh 60. The mesh serves to uniformly spread a sample such as blood and prevent generation of bubbles. Hemocytes are filtered from the supplied blood while the blood flows in a vertical direction within the filter 57. Hereat, a reagent present in the filter 57 acts to bring a selective enzyme reaction with LDL.

Thereafter, on arrival of the sample at the reagent dried body 53, cholesterol esterase, cholesterol oxidase, peroxidase, 4-aminoantipyrin and DAOS in the reagent dried body 53 act, resulting in coloring as in Embodiment 1. It is possible by the method thus described to measure LDL cholesterol in blood in a single step. According to the biosensor in accordance with the present embodiment, there is no need for separate addition of reagents in certain timings, and no need for maintenance.

### Embodiment 5

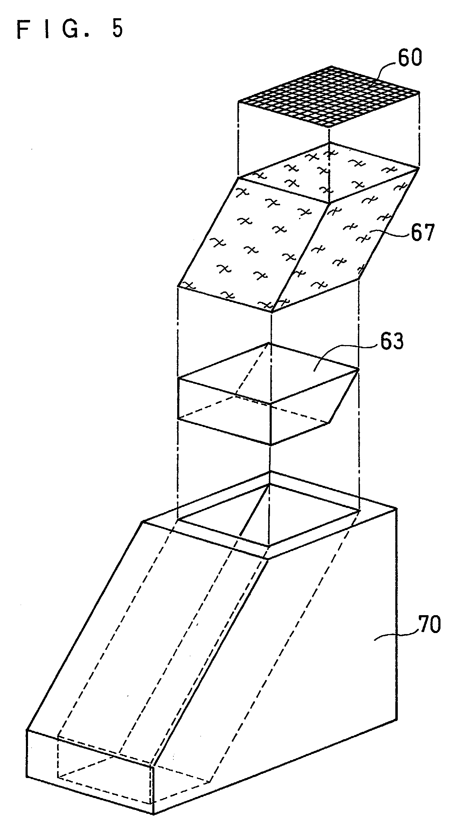

Next, FIG. 5 is an exploded oblique view of a biosensor in accordance with a fifth embodiment of the present invention. This biosensor is constituted of a container 70 having an oblique opening portion 70a, a reagent dried body 63, a filter 67 for filtering hemocytes from blood, and a mesh 60.

The reagent dried body 63 and the filter 67 are fitted in the oblique opening portion 70a penetrating the container 70, and the top face of the filter 67 is located as high as the top of the container 70, on which a mesh 60 is disposed.

In the case of using the biosensor having such a structure as shown in FIG. 5, a sample such as blood is dropped onto the mesh 60. The mesh serves to uniformly spread a sample such as blood and prevent generation of bubbles. Hemocytes are filtered from the supplied blood while the blood flows in an oblique direction within the filter 67. Hereat, a reagent present in the filter 67 acts to bring a selective enzyme reaction with LDL. Further, the container 70 is preferably made of a trans parent material such as glass or acrylic resin.

Thereafter, on arrival of the sample at the reagent dried body 63, cholesterol esterase, cholesterol oxidase, peroxidase, 4-aminoantipyrin and DAOS in the reagent dried body 63 act, resulting in coloring as in Embodiment 1. It is possible by the method thus described to measure LDL cholesterol in blood in a single step.

Having the flow channel oblique against the direction of gravity, the biosensor in accordance with the present embodiment has an advantage that a sample can be promptly filtered through the use of gravity and, further, how the sample is filtered can be readily observed. Since the reagent dried body 63 is located closest to the end, moreover, it is easy to confirm by visual observation as to whether the sample has reached the bottom of the container 70. The container 70 is preferably made of a transparent material such as glass, acrylic resin or polyethylene terephthalete.

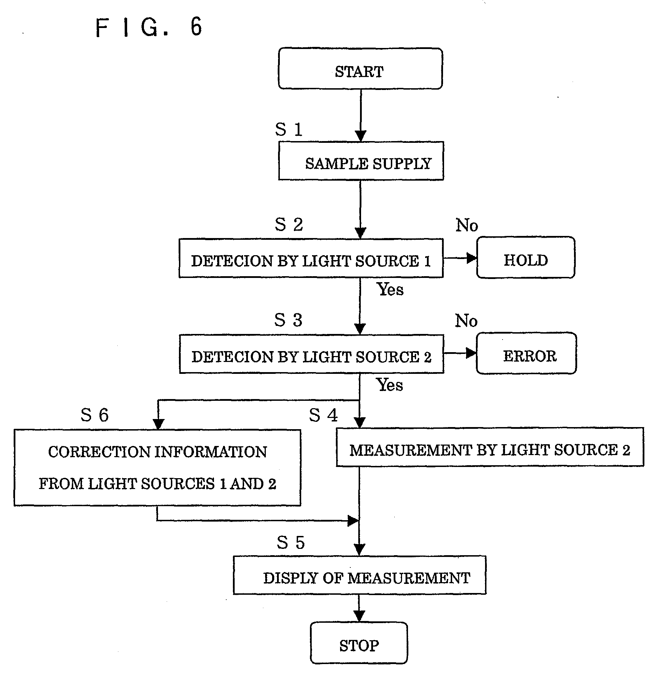

Herein, a usage method (determination method) of a test system using the biosensor of the present invention is described, as represented by the biosensor in accordance with the first embodiment shown in FIG. 1, with reference to the flowchart shown in FIG. 6.

First, a sample such as blood is supplied (Step 1). The test system is in HOLD status until the introduction of the sample is detected by a light source 1; once the sample introduction is detected (Step 2), it is then detected by a light source 2. If the sample introduction fails to be detected by the light source 2 (Step 3), an error results and the measurement is cancelled.

When the light source 2 detects the sample introduction (Step 3), it measures a degree of color (Step 4), determines LDL cholesterol, and displays a measurement result (Step 5). It should be noted that, if necessary, correction or compensation is conducted according to the correction or compensation information provided by the light source 1 or the light source 2 (Step 6), and then the measurement result is displayed (Step 5). Especially when the result is corrected by the use of the light source 2, the basis of the correction is on the information before and after the coloring.

It should be noted that, by simultaneous and continual measurements of a light in the hole 9 as a measurement portion and of a light in the hole 19 as a measurement portion, the time elapsed since the sample passes through the hole 19 (the light-source-1 side) until passing through the hole 9 (the light-source-2 side) can be measured. In the case where the flowing time of the sample exceeds a certain period of time, the measurement can be terminated by recognizing the sample detection as abnormal.

Moreover, the separation of the sample for bringing a selective enzyme reaction with LDL from cholesterol esterase, cholesterol oxidase and the like allows the reagent for bringing a selective enzyme reaction with LDL to be the first to act on the sample. Favorably, the sample can be sufficiently acted upon by the reagent for bringing a selective enzyme reaction with LDL before being subjected to the action of cholesterol esterase, cholesterol oxidase and the like. It should be noted that the sample supply portion 8 is preferably capable of holding a supplied sample for a certain period of time.

It is to be noted that the biosensor thus produced is preferably sealed to be stored in order to avoid absorption of moisture in the air. Storing the biosensor in the presence of an absorbent such as silica gel or alumina is even more preferred. Packing the biosensor with aluminum is also preferred as it can isolate lights and inhibit a reagent from deteriorating. Freeze-drying may also be used as a method for drying reagents. Water in a reagent is sufficiently removed by freeze-drying, to favorably inhibit deterioration in a reagent.

In the following, the present invention is specifically described using examples; the present invention is not limited thereto.

### Example 1

In the present example, a biosensor shown in FIG. 1 was fabricated and LDL cholesterol in blood was measured using blood as a sample.

First prepared was an assembly obtained by attaching by pressure a substrate 1 made of polyethylene terephthalate (PET), a PET-made transparent sheet 2 and a PET-made spacer 4. A filter 7 for filtering hemocytes from blood was provided in line with the right side of a slit 4a of the spacer 4. A glass filter was used as the filter 7. An aqueous solution containing a polyoxyethylene-polyoxypropylene copolymer as a reagent for bringing a selective enzyme reaction with LDL, α-cyclodextrin sulfate, dextran sulfate, and magnesium chloride was added dropwise into the filter 7 having been incorporated into the spacer 4, followed by drying.

Next, an aqueous solution containing cholesterol esterase, cholesterol oxidase, peroxidase, 4-aminoantipyrin, HDAOS and MOPS (pH 7.0) was dropped onto the transparent sheet 2, followed by drying to obtain a reagent dried body 3. Finally, a PET-made cover 5 was placed and thermally attached by pressure onto the spacer 4, to produce a dry-type test strip in accordance with the present invention.

Blood was dropped onto the vicinity of a sample supply portion 8. Hemocytes were filtered from the supplied blood while the blood flew in a horizontal direction within the filter 7. The filtered blood reached the vicinity of an air vent 6 to dissolve the reagent dried body 3. At this time, the reagent for bringing a selective enzyme reaction with LDL, which was present in the filter 7, and cholesterol esterase, cholesterol oxidase, peroxidase, 4-aminoantipyrin and HDAOS in the reagent dried body 3 acted, resulting in coloring.

The degree of the color was measured by irradiating a light, emitted by a light-emitting diode, from the under part of the substrate 1 through a hole 9 as a measurement portion, and then detecting a reflected light by a photodiode. It was possible by the method thus described to conduct a one-step measurement of LDL cholesterol in blood by only a one-time supply of blood.

### Example 2

In the present example, a biosensor shown in FIG. 2 was fabricated and LDL cholesterol in blood was measured using blood as a sample.

First prepared was an assembly obtained by fixing, with an adhesive agent, a PET-made substrate 11, a PET-made transparent sheet 12 and a PET-made spacer 14. An aqueous solution containing cholesterol esterase, cholesterol oxidase, peroxidase, 4-aminoantipyrin, HDAOS and MOPS (pH 7.0) was dropped onto the transparent sheet 12, followed by drying to obtain a reagent dried body 13.

Thereon fixed with an adherent agent were a PET-made cover 15, a PET-made spacer 24 and a PET-made cover 25, and a filter 17 for filtering hemocytes from blood was inserted from a sample supply portion 18. Finally, an aqueous solution containing a polyoxyehylene-polyoxypropylene copolymer, α-cyclodextrin sulfate, dextran sulfate, and magnesium chloride was dropped into the filter 17, followed by drying.

Blood was dropped into the sample supply portion 18. Being in mortar shape, the sample supply portion 18 helped with a smooth sample supply. Hereat, the reagent present in the filter 17 acted to bring a selective enzyme reaction with LDL. In the biosensor, the filtered blood flew horizontally to the vicinity of the air vent 16, to dissolve the reagent dried body 13.

At this time, the reagent for bringing a selective enzyme reaction with LDL, which was present in the filter 17, and cholesterol esterase, cholesterol oxidase, peroxidase, 4-aminoantipyrin and HDAOS in the reagent dried body 13 acted, resulting in coloring as in Embodiment 1. It was possible by the method thus described to measure LDL cholesterol in blood in a single step.

### Example 3

In the present example, a biosensor shown in FIG. 3 was produced and LDL cholesterol in blood was measured using blood as a sample.

First prepared was an assembly obtained by thermally attaching by pressure a PET-made spacer 34 and a PET-made transparent sheet 32. An aqueous solution containing lipoprotein lipase, cholesterol esterase, cholesterol oxidase, peroxidase, 4-aminoantipyrin, DAOS and MOPS (pH 7.0) was dropped into a hole 34a of the spacer 34, followed by drying to obtain a reagent dried body 43.

Thereon placed was a filter 47. A polyoxyehylene-polyoxypropylene copolymer, α-cyclodextrin sulfate, dextran sulfate, and magnesium chloride were placed on the filter 47 as the pellet-like reagent dried body 33. Finally, a filter 37 was placed thereon to produce a biosensor in accordance with the present invention. A glass filter was used for the filter 37 as well as for the filter 47.

Blood was dropped into the filter 37. Hemocytes were filtered from the supplied blood when the blood flew in a vertical direction within the filter 34 or the filter 47. Hereat, the reagent dried body 43 acted to bring a selective enzyme reaction with LDL. The biosensor had the advantage of reducing the measurement time since the blood flew vertically within the filter through the use of gravity. It was possible by the method thus described to measure LDL cholesterol in blood in a single step.

Since the reagent for bringing a selective enzyme reaction with LDL was separated, via the filter 47, from enzymes such as cholesterol esterase and cholesterol oxidase, the reagent for bringing a selective enzyme reaction with LDL could be the first to act on the sample. Further, the sample could be sufficiently acted upon by the reagent for bringing a selective enzyme reaction with LDL before being subjected to the action of enzymes such as cholesterol esterase and cholesterol oxidase.

### Example 4

In the present example, a biosensor shown in FIG. 4 was produced and LDL cholesterol in blood was measured using blood as a sample.

First prepared was an assembly obtained by fixing, with an adhesive agent, a PET-made substrate 51, a PET-made transparent sheet 52 and a PET-made spacer 54. An aqueous solution containing cholesterol esterase, cholesterol oxidase, peroxidase, 4-aminoantipyrin, DAOS and MOPS (pH 7.0) was dropped onto the transparent sheet 52, followed by drying to obtain a reagent dried body 53.

Next, an aqueous solution containing a polyoxyehylene-polyoxypropylene copolymer, α-cyclodextrin sulfate, dextran sulfate, and magnesium chloride was dropped into a filter 57, which was dried and then placed on the reagent dried body 53. Finally a mesh 60 was fixed with an adhesive agent to produce a biosensor in accordance with the present invention.

Blood was dropped onto the mesh 60. The mesh served to uniformly spread the blood and prevent generation of bubbles.

Hemocytes were filtered from the supplied blood while the blood flew in a vertical direction within the filter 57. Hereat, the reagent present in the filter 57 acted to bring a selective enzyme reaction with LDL. Thereafter, on arrival of the blood at the reagent dried body 53, cholesterol esterase, cholesterol oxidase, peroxidase, 4-aminoantipyrin and DAOS in the reagent dried body 53 acted, resulting in coloring as in Embodiment 1. It was possible by the method thus described to measure LDL cholesterol in blood in a single step.

### Example 5

In the present example, a biosensor shown in FIG. 5 was produced and LDL cholesterol in blood was measured using blood as a sample.

First, an aqueous solution containing cholesterol esterase, cholesterol oxidase, peroxidase, 4-aminoantipyrin, DAOS and MOPS (pH 7.0) was dropped onto the bottom of a container 70 with an oblique opening, followed by drying to obtain a reagent dried body 63.

Next, an aqueous solution containing a polyoxyehylene-polyoxypropylene copolymer, α-cyclodextrin sulfate, dextran sulfate, and magnesium chloride was dropped into a filter 67, which was dried and then placed on the reagent dried body 63. Finally a mesh 60 was fixed with an adhesive agent to the container 70 to produce a biosensor in accordance with the present invention.

Blood was dropped onto the mesh 60. The mesh served to uniformly spread a sample such as blood and prevent generation of bubbles. Hemocytes were filtered from the supplied blood while the blood flew in an oblique direction within the filter 67. Hereat, the reagent present in the filter 67 acted to bring a selective enzyme reaction with LDL. Thereafter, on arrival of the blood at the reagent dried body 63, cholesterol esterase, cholesterol oxidase, peroxidase, 4-aminoantipyrin and DAOS in the reagent dried body 63 acted, resulting in coloring as in Embodiment 1. It was possible by the method thus described to measure LDL cholesterol in blood in a single step.

### Industrial Applicability

The biosensor in accordance with the present invention is capable of simply determining cholesterol contained in low-density lipoprotein (LDL) in a sample such as blood, serum and plasma, and it can thus be used favorably in diagnoses of high cholesterol thrombus at home and hospitals.

## Claims

1. A biosensor comprising:
a substrate; and
cholesterol esterase, cholesterol oxidase or cholesterol dehydrogenase, a reagent for bringing a selective enzyme reaction with low-density lipoprotein, peroxidase, and a dye source, which are carried on said substrate.

2. The biosensor in accordance with claim 1, further comprising a carrier on said substrate,
said carrier being made of a paper filter, a glass filter, a membrane filter or a cellulose fiber.

3. The biosensor in accordance with claim 2, wherein said cholesterol esterase, said cholesterol oxidase or cholesterol dehydrogenase, said reagent, said peroxidase, and said dye source are carried on said carrier by freeze drying, high-temperature drying, hot-air drying or natural drying.

4. The biosensor in accordance with claim 3, wherein said reagent is carried as being separated from said cholesterol esterase, said cholesterol oxidase or cholesterol dehydrogenase, and said dye source.

5. The biosensor in accordance with claim 4, further comprising a flow channel,
said reagent being provided upstream of said flow channel, while said cholesterol esterase, said cholesterol oxidase or cholesterol dehydrogenase, said peroxidase, and said dye source being provided downstream of said flow channel.

6. The biosensor in accordance with claim 1, wherein said reagent is a surfactant.

7. The biosensor in accordance with claim 6, wherein said surfactant is either a nonion-type surfactant or an anion-type surfactant.

8. The biosensor in accordance with claim 7, wherein said nonion-type surfactant is a nonion-type polymer.

9. The biosensor in accordance with claim 1, further comprising α-cyclodextrin sulfate.

10. The biosensor in accordance with claim 1, further comprising dextran sulfate.

11. The biosensor in accordance with claim 1, further comprising a pH buffer.

12. The biosensor in accordance with claim 1, further comprising a bivalent metal salt.

13. The biosensor in accordance with claim 12, wherein said bivalent metal salt is any of magnesium salt, calcium salt, and manganese salt.

14. The biosensor in accordance with claim 1, further comprising lipoprotein lipase.

15. The biosensor in accordance with claim 1, further comprising a filter for separating a specific element from a sample.

16. The biosensor in accordance with claim 15, wherein the passage of said sample through said filter and gravity are parallel, vertical or oblique in direction.

17. A test system including a first light source and a biosensor comprising:
a substrate, and
cholesterol esterase, cholesterol oxidase or cholesterol dehydrogenase, a reagent for bringing a selective enzyme reaction with low-density lipoprotein, peroxidase, and a dye source, which are carried on said substrate.

18. The test system in accordance with claim 17, further including a second light source.
